# EUROPEAN PATENT APPLICATION

(11) **EP 4 811 326 A1**
(43) Date of publication of application: **23.09.2026**
(21) Application number: 25773996.1
(22) Date of filing: 23.01.2025
(51) Int. Cl.: G08B 21/02, G01S 13/34

(54) **FALL DETECTING DEVICE AND FALL DETECTING METHOD**

(30) Priority: 19.03.2024 JP 2024044059
(71) Applicant: Murata Manufacturing Co., Ltd., Nagaokakyo-shi, Kyoto 617-8555 (JP)
(72) Inventor: YAMAGUCHI, Kenta, Nagaokakyo-shi, Kyoto 617-8555 (JP); ASANO, Kosei, Nagaokakyo-shi, Kyoto 617-8555 (JP); KINOSHITA, Takahiro, Nagaokakyo-shi, Kyoto 617-8555 (JP)
(74) Representative: Stöckeler, Ferdinand
(86) International application number: PCT/JP2025/002110
(87) International publication number: WO 2025/197292

(57) **Abstract**

A fall detector and a fall detection method by which computational load is reduced are provided. The fall detector includes a sensor; a signal strength calculator that calculates respective signal strengths of a plurality of range bins in a detection target area, based on transmitted and received signals of the sensor; a signal strength variation calculator that calculates respective signal strength variations of the range bins; an area setter that sets a first area in the detection target area, the first area not including a range bin of the range bins that has a corresponding one of the signal strengths that is greater than or equal to a signal strength threshold; and a determiner that detects an occurrence of a fall of a living body in the detection target area based on a first signal strength variation serving as a statistical value of a plurality of signal strength variations of a plurality of range bins in the first area.

## Description

### Technical Field

The present invention relates to a fall detector and a fall detection method.

### Background Art

There is an issue of accidents or falls in an individual room in various scenes. For example, inmates in a nursing facility spend a long time in the individual room, and particularly elderly people and people who require nursing care are in circumstances where they are likely to fall due to deterioration of bodily functions or changes in cognitive function. In a factory, a worker sometimes works alone in an individual room for a long time, and there is a risk of the occurrence of a fall or an unexpected accident during the work. In addition, even a room other than an individual room used by a single person or a worker alone possibly has the same risk if the fall or the unexpected accident occurs in a state where another person is absent. If such a fall accident occurs, early detection and quick treatment are required. However, a case where a monitoring device such as a camera is difficult to install in the room due to consideration of privacy or the like leads to concern about a delay in anomaly detection or treatment. Accordingly, to date, by tracking the movement of a living body with a radar, the anomaly state has been found based on the detected location or state of the living body (for example, Patent Document 1).

Specifically, Patent Document 1 discloses a determination of an anomaly state of a living body, the determination being made in a case where time variation in a signal strength greater than that under normal conditions occurs in a target-present area (target-present range bin) where a measurement target is present or in a case where a living body is in a stationary state continuously for a predetermined time period.

### Citation List

### Patent Document

Patent Document 1: Japanese Unexamined Patent Application Publication No. 2020-081312

### Summary of Invention

### Technical Problem

In Patent Document 1 described above, determination of whether a measurement target is present is performed for each of a plurality of areas (range bins) into which a detection area is divided in a direction corresponding to a distance to and from the detection area. Accordingly, there is a possibility of a high computational load on a processor.

The present disclosure has been made under the circumstances above, and it is an object thereof to provide a fall detector and a fall detection method by which a computational load is reduced.

### Solution to Problem

A fall detector according to an aspect of the present disclosure includes a sensor; a signal strength calculator that calculates respective signal strengths of a plurality of range bins in a detection target area, based on transmitted and received signals of the sensor; a signal strength variation calculator that calculates respective signal strength variations of the range bins; an area setter that sets a first area in the detection target area, the first area not including a range bin of the range bins that has a corresponding one of the signal strengths that is greater than or equal to a signal strength threshold; and a determiner that detects an occurrence of a fall of a living body in the detection target area based on a first signal strength variation serving as a statistical value of respective signal strength variations of a plurality of range bins in the first area.

With this configuration, the first area that does not include the range bin where the signal strength is greater than or equal to the signal strength threshold is set, and the occurrence of the fall of the living body in the detection target area is detected based on the first signal strength variation serving as the statistical value of the respective signal strength variations of the plurality of range bins in the first area. The computational load may thereby be reduced.

A fall detection method according to an aspect of the present disclosure includes calculating respective signal strengths of a plurality of range bins in a detection target area, based on transmitted and received signals of a sensor; calculating respective signal strength variations of the range bins; setting a first area in the detection target area, the first area not including a range bin of the range bins that has a corresponding one of the signal strengths that is greater than or equal to a signal strength threshold; and detecting an occurrence of a fall of a living body in the detection target area, based on a first signal strength variation serving as a statistical value of respective signal strength variations of a plurality of range bins in the first area.

With this configuration, the first area that does not include the range bin where the signal strength is greater than or equal to the signal strength threshold is set, and the occurrence of the fall of the living body in the detection target area is detected based on the statistical value of the respective signal strength variations of the plurality of range bins in the first area. The computational load may thereby be reduced.

### Advantageous Effects of Invention

According to the present disclosure, the fall detector and the fall detection method by which the computational load is reduced may be provided.

### Brief Description of Drawings

[Fig. 1] Fig. 1 is a block diagram illustrating the schematic configuration of a fall detector.
[Fig. 2] Fig. 2 is a schematic layout of an individual room where the fall detector is installed, the individual room being viewed from above.
[Fig. 3] Fig. 3 is a view illustrating an example of a detection target area.
[Fig. 4] Fig. 4 is a view illustrating an example of area setting in a case where the detection target area is inside the individual room.
[Fig. 5] Fig. 5 is a view illustrating an example of area setting in a case where the detection target area is inside a sick room.
[Fig. 6] Fig. 6 is a flowchart illustrating a specific example of a fall detection process.
[Fig. 7] Fig. 7 is a block diagram illustrating an example of a fall detection system.

### Description of Embodiments

Hereinafter, a fall detector and a fall detection method according to an embodiment will be described in detail based on the drawings. Note that the embodiment does not limit the present disclosure.

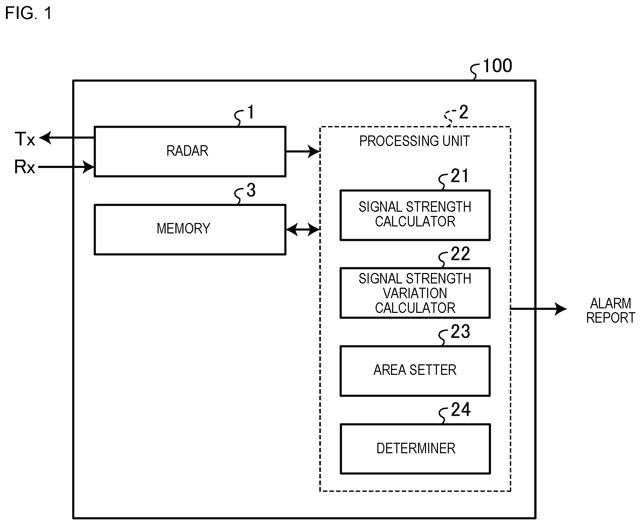

Fig. 1 is a block diagram illustrating the schematic configuration of a fall detector. A fall detector 100 according to the embodiment includes a radar 1, a processing unit 2, and a memory 3.

In the configuration of the fall detector 100 according to the embodiment, the radar 1 is a radar based on, for example, a frequency modulated continuous wave (FMCW) method or a fast chirp modulation (FCM) method. The radar based on the FMCW method and the FCM method is known, and thus detailed explanation is omitted on occasions. The present disclosure is widely applicable to a sensor capable of presuming location information regarding a target by using an electromagnetic wave (including light) or an acoustic wave.

The processing unit 2 is a component implementable by the processing of an arithmetic processing unit such as a central processing unit (CPU) or a micro processing unit (MPU). The memory 3 is composed of a memory device such as a read only memory (ROM) or a random access memory (RAM).

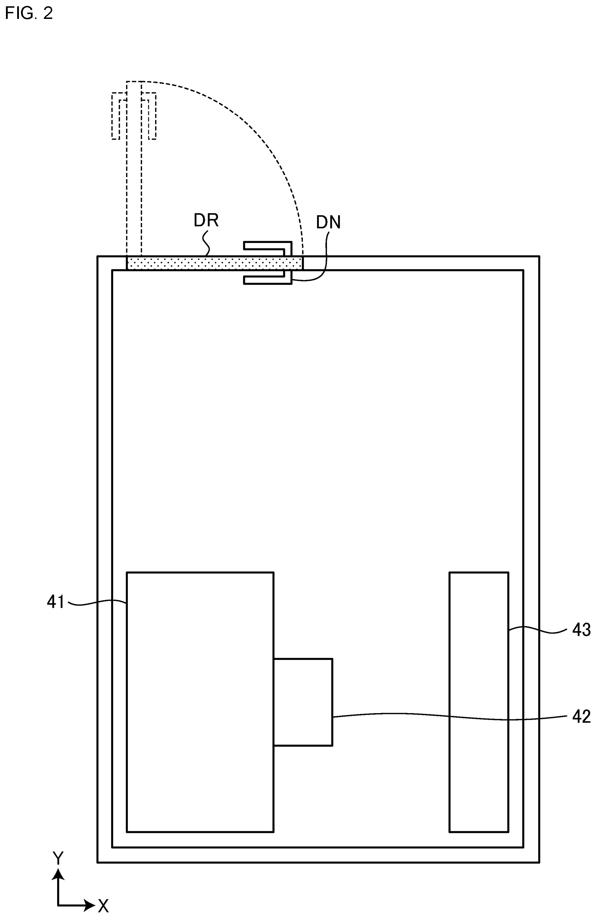

Fig. 2 is a schematic layout of an individual room where the fall detector is installed, the individual room being viewed from above. In the example illustrated in Fig. 2, the fall detector 100 according to the embodiment is configured and disposed to detect a fall of a person in the individual room.

The individual room where the fall detector 100 is installed has a door DR to be opened toward the outside. In the example illustrated in Fig. 2, the individual room also has a desk 41, a chair 42, and a shelf 43 therein. The location of the door DR provided in the individual room and the type and the layout of furniture or the like provided inside the individual room are not limited to the example illustrated in Fig. 2.

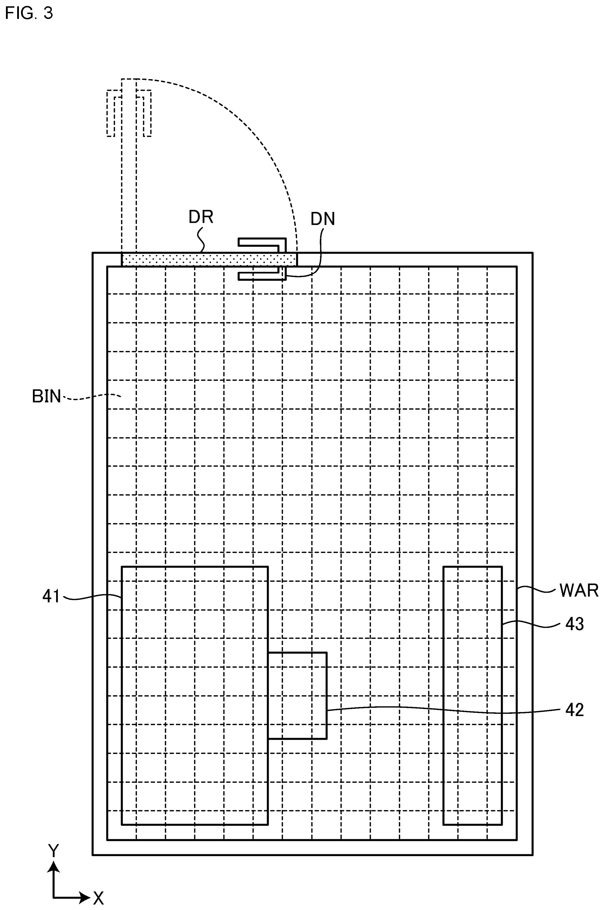

Fig. 3 is a view illustrating an example of the detection target area. Fig. 3 illustrates an example in which an area inside the individual room illustrated in Fig. 2 serves as a detection target area WAR of the fall detector 100.

It suffices that the radar 1 be installed in a location where transmitted and received signals enabling extraction of respective signals of a plurality of range bins BIN are acquired, the plurality of range bins BIN being obtained by dividing the detection target area WAR in a sight line direction illustrated in Fig. 3 (a Y direction) and a direction (an X direction) orthogonal to the sight line direction, the Y direction serving as the sight line direction of the radar 1. Fig. 3 illustrates an example of defining the plurality of range bins BIN based on the XY directions orthogonal to each other; however, the technology according to the present disclosure may be applied in such a manner that a direction (R direction) corresponding to a distance to and from the radar 1 and an angular direction (θ direction) are defined.

Specifically, the radar 1 may be installed, for example, on the wall surface or the ceiling of the individual room, and may be installed, for example, on a structural member or the like of furniture (the desk 41, the chair 42, or the shelf 43) provided inside the individual room. Furthermore, the radar 1 or the fall detector 100 including the radar 1 may be disposed at any location inside an existing individual room. The installation location or the arrangement location of the radar 1 does not limit the present disclosure.

The width of each range bin BIN is, for example, 10 cm or shorter. The number of range bins BIN in the detection target area WAR may be changed appropriately based on the width of the range bin BIN or the size of the detection target area WAR.

The processing unit 2 includes a signal strength calculator 21, a signal strength variation calculator 22, an area setter 23, and a determiner 24.

The signal strength calculator 21 calculates respective signal strengths Pbin of the plurality of range bins BIN in the detection target area WAR based on the transmitted and received signals of the radar 1. The signal strength calculator 21 stores the calculated respective signal strengths Pbin of the plurality of range bins BIN in the memory 3.

The signal strength variation calculator 22 calculates respective signal strength variations PVbin of the plurality of range bins BIN in the detection target area WAR. The signal strength variation calculator 22 stores the calculated signal strength variations PVbin of the plurality of range bins BIN in the memory 3.

The signal strength variation PVbin represents the temporal variation in the signal strength Pbin of each of the plurality of range bins BIN. If a living body (moving body) is not present in the detection target area WAR, the signal strength variation PVbin of each of the plurality of range bins BIN in the detection target area WAR is substantially zero.

The area setter 23 sets an area for determining whether the living body is present in a fall detection process (described later), based on the signal strength calculated by the signal strength calculator 21. Hereinafter, a process for determining whether a living body is present is also referred to as a living-body-presence determination process.

Specifically, the area setter 23 sets, as a first area PAR1, an area that does not include a range bin where the signal strength Pbin is greater than or equal to a signal strength threshold in the detection target area WAR, and sets, as a second area PAR2, an area excluding the first area PAR1. Hereinafter, a process for setting the first area PAR1 and the second area PAR2 is also referred to as an area setting process. The signal strength threshold used in the area setting process is set as a threshold for determining whether a stationary object is present in one of the range bins BIN in the detection target area WAR. The signal strength threshold may be a constant value uniformly used in the detection target area WAR or may be a variation value that varies with a distance from the radar 1. Alternatively, the threshold determination may be performed by using an adaptive algorithm such as a constant false alarm rate (CFAR).

The area setting process by the area setter 23 is executed in a state where the living body is not present in the detection target area WAR. In other words, the area setter 23 executes the area setting process in a case where the signal strength variation PVbin of each of the plurality of range bins BIN in the detection target area WAR is substantially zero (specifically, a very small value smaller than or equal to the noise floor for the processing unit 2).

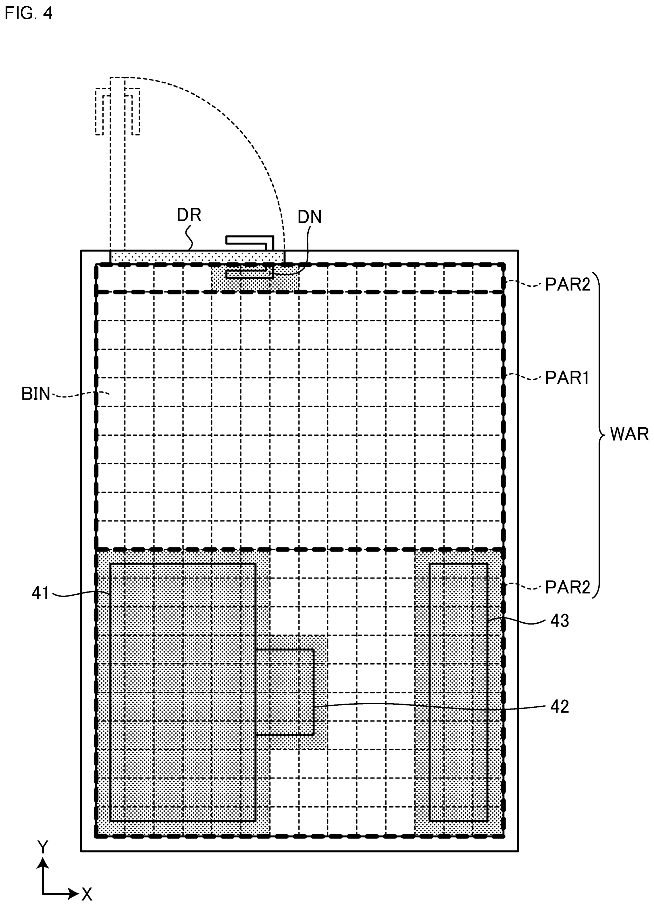

Fig. 4 is a view illustrating an example of area setting in a case where the detection target area is inside the individual room. In the example illustrated in Fig. 4, the range bins BIN each having the signal strength Pbin exceeding the signal strength threshold are hatched. Fig. 4 illustrates an example of the signal strength Pbin exceeding the signal strength threshold in the range bins BIN including the furniture (the desk 41, the chair 42, and the shelf 43) provided inside the individual room and a doorknob DN of the door DR. In the present disclosure, as described above, an area that does not include a range bin BIN having the signal strength Pbin exceeding the signal strength threshold is set as the first area PAR1, and an area excluding the first area PAR1 is set as the second area PAR2.

In the fall detector 100 according to the embodiment, the first area PAR1 is an area where a person is assumed not to lie under normal conditions.

Specifically, in the example illustrated in Fig. 4, for example, when a person faints and falls due to an acute disease, the person who has fallen on the flow line between the door DR and the furniture (the desk 41, the chair 42, or the shelf 43) is assumed to come to be in a lying state. In this case, a space including a space on the flow line between the door DR and the furniture (the desk 41, the chair 42, or the shelf 43) is set as the first area PAR1, and spaces including the furniture (the desk 41, the chair 42, and the shelf 43) and the doorknob DN of the door DR are each set as the second area PAR2.

The individual room serving as the detection target area WAR of the fall detector 100 according to the embodiment is not limited to what is called a private room in a typical dwelling, and examples thereof include single-occupancy rooms in nursing facilities, individual work booths and individual-room-type work spaces in business establishments such as factories and warehouses, bathrooms, changing rooms, and toilet rooms.

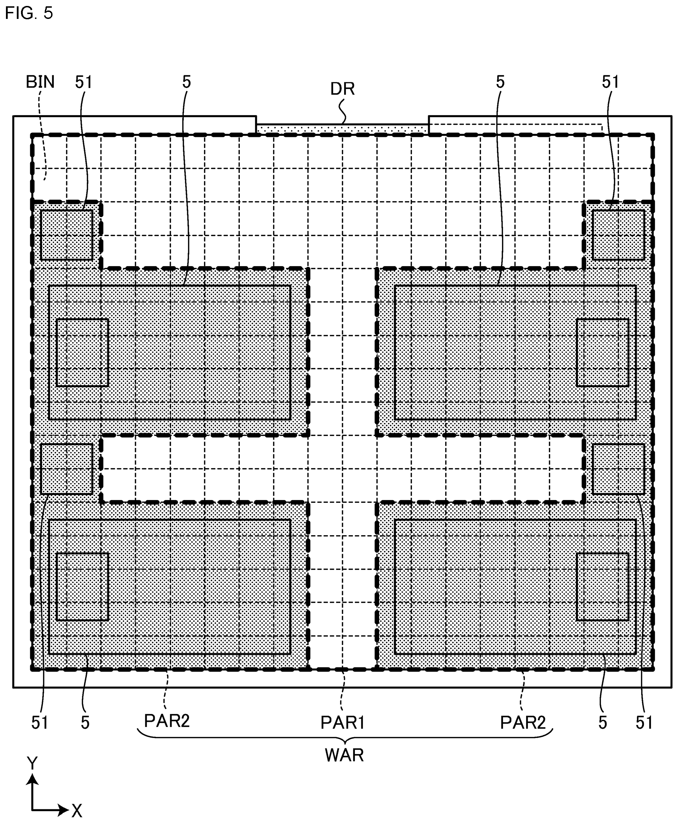

In addition, for example, a sick room or the like usable by persons may serve as the detection target area WAR of the fall detector 100. Fig. 5 is a view illustrating an example of area setting in a case where a detection target area is inside a sick room. In the example illustrated in Fig. 5, the fall detector 100 is configured to detect a fall of a person in the sick room. In the example illustrated in Fig. 5, the schematic layout of the sick room where the fall detector 100 is installed is illustrated, the sick room being viewed from above. An example where an area inside the sick room serves as the detection target area WAR for the fall detector 100 is also illustrated.

In the example illustrated in Fig. 5, like the example illustrated in Fig. 4, the range bins BIN each having the signal strength Pbin exceeding the signal strength threshold are hatched. Fig. 5 illustrates an example of the signal strength Pbin exceeding the signal strength threshold in the range bins BIN including beds 5 and bedside cupboards 51.

In the example illustrated in Fig. 5, for example, when a patient falls off the bed 5, the patient having fallen beside one of the beds 5 is assumed to be in the lying state. In this case, an area including at least a space beside the bed 5 is set as the first area PAR1, and a space including the bed 5 and the bedside cupboard 51 is set as the second area PAR2.

Specifically, it suffices that the first area PAR1 include, for example, in a two-dimensional space on the XY plane defined based on the X direction and the Y direction, at least a space in which range bins BIN each having the signal strength Pbin not exceeding the signal strength threshold extend continuously in a first direction by a first length (for example, 60 cm) and in a second direction orthogonal to the first direction by a second length (for example, 40 cm).

In the examples illustrated in Fig. 4 and Fig. 5, the area setting performed on the two-dimensional space on the XY plane has been exemplified; however, the area setting may be performed on a three-dimensional space including a Z direction (for example, the individual room orthogonal to the XY plane and a height direction of the sick room). A space under the desk 41 provided inside the individual room illustrated in Fig. 4 and a space above the floor surface and under the bed 5 in the sick room illustrated in Fig. 5 may thereby be set as the first area PAR1. Alternatively, a space with a height greater than or equal to a predetermined value may be set as the second area PAR2.

In this case, it suffices that the first area PAR1 include, for example, at least a space in which the range bins BIN each having the signal strength Pbin not exceeding the signal strength threshold extend continuously on the XY plane in the first direction by the first length (for example, 60 cm) and in the second direction orthogonal to the first direction by the second length (for example, 40 cm) and with a third length (for example, 20 cm) from the floor surface in the Z direction orthogonal to the XY plane.

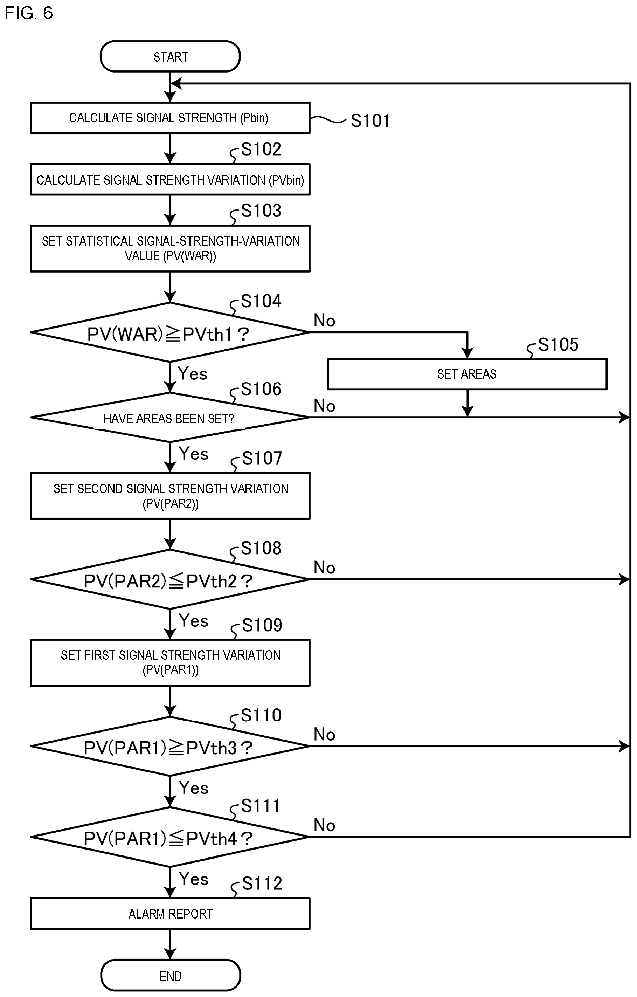

The determiner 24 is a component that executes various determination processes in the fall detection process according to the embodiment described below, the determination processes including the process for determining the presence of a living body in the detection target area WAR, the process for determining the presence of a living body in the first area PAR1, and the process for determining the presence of a living body in the second area PAR2. Hereinafter, a specific example of a fall detection process according to the embodiment will be described. Fig. 6 is a flowchart illustrating the specific example of the fall detection process. The fall detection process illustrated in Fig. 6 is executed, for example, in frame periods in which the radar 1 acquires an IF signal.

The signal strength calculator 21 calculates the signal strength Pbin of each of the plurality of range bins BIN in the detection target area WAR based on the transmitted and received signals of the radar 1 (step S101). The signal strength calculator 21 stores the calculated signal strength Pbin of each of the plurality of range bins BIN in the memory 3.

The signal strength variation calculator 22 calculates the signal strength variation PVbin of each of the plurality of range bins BIN in the detection target area WAR (step S102). Specifically, the signal strength variation calculator 22 calculates a distribution or a standard deviation of the signal strengths in a plurality of frames as the signal strength variation PVbin of each of the plurality of range bins BIN. Alternatively, for each of the plurality of range bins BIN in the detection target area WAR, a difference between the signal strength Pbin(t) at time t and the signal strength Pbin(t-) at time t- earlier than the time t may be calculated as the signal strength variation PVbin of each of the plurality of range bins BIN.

The determiner 24 sets, as a statistical signal-strength-variation value PV(WAR), the maximum value of the respective signal strength variations PVbin of the plurality of range bins BIN in the detection target area WAR read out from the memory 3 (step S103) and determines whether the statistical signal-strength-variation value PV(WAR) is greater than or equal to a first threshold PVth1 (step S104).

The first threshold PVth1 is a determination threshold for determining whether a living body is present in the detection target area WAR and is stored in advance in the memory 3. A very small value in consideration of the noise floor for the processing unit 2 is set as the first threshold PVth1. The statistical signal-strength-variation value PV(WAR) is not limited to the maximum value of the respective signal strength variations PVbin of the plurality of range bins BIN in the detection target area WAR. Specifically, the statistical signal-strength-variation value PV(WAR) may be, for example, the mean value of the respective signal strength variations PVbin of the plurality of range bins BIN in the detection target area WAR.

If the statistical signal-strength-variation value PV(WAR) is less than the first threshold PVth1 (No in step S104), the determiner 24 determines that a living body is not present in the detection target area WAR.

If the determiner 24 determines that a living body is not present in the detection target area WAR (No in step S104), the area setter 23 sets, as the first area PAR1, an area that does not include a range bin where the signal strength Pbin is greater than or equal to the signal strength threshold in the detection target area WAR, and sets, as the second area PAR2, an area excluding the first area PAR1, as described above (step S105). The area setter 23 stores the first area PAR1 and the range bins BIN included in the first area PAR1 in the memory 3 in association with each other. The area setter 23 also stores the second area PAR2 and the range bins BIN included in the second area PAR2 in the memory 3 in association with each other.

If the setting of the first area PAR1 and the second area PAR2 (step S105) is completed, the process returns to step S101, and step S101 and the following steps are performed in the next frame period.

If the statistical signal-strength-variation value PV(WAR) is greater than or equal to the first threshold PVth1 (Yes in step S104), the determiner 24 determines that a living body is present in the detection target area WAR.

If the determiner 24 determines that a living body is present in the detection target area WAR (Yes in step S104), the determiner 24 subsequently determines whether the first area PAR1 and the second area PAR2 have been set (step S106).

If the first area PAR1 and the second area PAR2 have not been set (No in step S106), the process returns to step S101, and step S101 and the following steps are performed in the next frame period.

If the first area PAR1 and the second area PAR2 have been set (Yes in step S106), the determiner 24 subsequently sets, as a second signal strength variation PV(PAR2), the maximum value of respective signal strength variations PVbin of a plurality of range bins BIN in the second area PAR2 read out from the memory 3 (step S107) and determines whether the second signal strength variation PV(PAR2) is less than or equal to a second threshold PVth2 (step S108).

The second threshold PVth2 is a determination threshold for determining whether a living body is present in the second area PAR2 and is stored in advance in the memory 3. The second signal strength variation PV(PAR2) is not limited to the maximum value of the respective signal strength variations PVbin of the plurality of range bins BIN in the second area PAR2. Specifically, the second signal strength variation PV(PAR2) may be, for example, the mean value of the respective signal strength variations PVbin of the plurality of range bins BIN in the second area PAR2.

If the statistical signal-strength-variation value PV(WAR) of the detection target area WAR and the second signal strength variation PV(PAR2) of the second area PAR2 (for example, the maximum value of the respective signal strength variations PVbin of the plurality of range bins BIN) have the same statistical value, the first threshold PVth1 for determining that a living body is present in the detection target area WAR and the second threshold PVth2 for determining that a living body is present in the second area PAR2 may have the same value.

If the second signal strength variation PV(PAR2) is greater than the second threshold PVth2 (No in step S108), the determiner 24 determines that a living body is present in the second area PAR2.

The presence of the living body in the second area PAR2 in the example illustrated in Fig. 4 leads to an assumption that, for example, a person is sitting on the chair 42 in the individual room. In addition, the presence of the living body in the second area PAR2 in the example illustrated in Fig. 5 leads to an assumption that, for example, a patient lies on the bed 5 in the sick room.

If the determiner 24 determines that a living body is present in the second area PAR2 (No in step S108), the process returns to step S101, and step S101 and the following steps are performed in the next frame period.

If the second signal strength variation PV(PAR2) is less than or equal to the second threshold PVth2 (Yes in step S108), the determiner 24 subsequently sets, as a first signal strength variation PV(PAR1), the maximum value of respective signal strength variations PVbin of a plurality of range bins BIN in the first area PAR1 read out from the memory 3 (step S109).

The first signal strength variation PV(PAR1) is not limited to the maximum value of the respective signal strength variations PVbin of the plurality of range bins BIN in the first area PAR1. Specifically, the first signal strength variation PV(PAR1) may be, for example, the mean value of the respective signal strength variations PVbin of the plurality of range bins BIN in the first area PAR1.

In the example illustrated in Fig. 4, for example, if a person is standing on the flow line between the door DR and the furniture (the desk 41, the chair 42, or the shelf 43) in the individual room, the first signal strength variation PV(PAR1) is assumed to become higher than in another state. In addition, in the example illustrated in Fig. 5, for example, if a patient, a doctor, or a nurse is standing beside the bed 5 in the sick room, the first signal strength variation PV(PAR1) is assumed to become higher. In other words, if the living body present in the detection target area WAR does not have an anomaly, the first signal strength variation PV(PAR1) is assumed to become higher.

In contrast, in the example illustrated in Fig. 4, for example, if a person faints and falls due to an acute disease in the individual room and lies on the flow line between the door DR and the furniture (the desk 41, the chair 42, or the shelf 43), the first signal strength variation PV(PAR1) is assumed to become lower. In addition, in the example illustrated in Fig. 5, for example, if the patient falls off the bed 5 and lies beside the bed 5 in the sick room, the first signal strength variation PV(PAR1) is assumed to become lower. In other words, if the living body present in the detection target area WAR has an anomaly, the first signal strength variation PV(PAR1) is assumed to become lower.

The determiner 24 determines whether the first signal strength variation PV(PAR1) set in step S109 is greater than or equal to a third threshold PVth3 (step S110).

The third threshold PVth3 is a determination threshold for determining whether a living body is present in the first area PAR1 and is stored in advance in the memory 3. If the statistical signal-strength-variation value PV(WAR) of the detection target area WAR and the first signal strength variation PV(PAR1) of the first area PAR1 (for example, the maximum value of the respective signal strength variations PVbin of the plurality of range bins BIN) have the same statistical value, the first threshold PVth1 for determining that a living body is present in the detection target area WAR and the third threshold PVth3 for determining that a living body is present in the first area PAR1 may have the same value.

If the second signal strength variation PV(PAR2) of the second area PAR2 and the first signal strength variation PV(PAR1) of the first area PAR1 (for example, the maximum value of the respective signal strength variations PVbin of the plurality of range bins BIN) have the same statistical value, the second threshold PVth2 for determining that a living body is present in the second area PAR2 and the third threshold PVth3 for determining that a living body is present in the first area PAR1 may have the same value.

If the first signal strength variation PV(PAR1) is less than the third threshold PVth3 (No in step S110), the process returns to step S101, and step S101 and the following steps are performed in the next frame period.

If the first signal strength variation PV(PAR1) is greater than or equal to the third threshold PVth3 (Yes in step S110), the determiner 24 subsequently determines whether the first signal strength variation PV(PAR1) set in step S109 is less than or equal to a fourth threshold PVth4 (step S111).

The fourth threshold PVth4 is a determination threshold for determining whether the living body present in the detection target area WAR has an anomaly and is stored in advance in the memory 3. The fourth threshold PVth4 has a greater value than that of the third threshold PVth3 for determining whether a living body is present in the first area PAR1.

If the first signal strength variation PV(PAR1) is greater than the fourth threshold PVth4 (No in step S111), the process returns to step S101, and step S101 and the following steps are performed in the next frame period.

If the first signal strength variation PV(PAR1) is less than or equal to the fourth threshold PVth4 (Yes in step S111), the determiner 24 determines that the living body present in the detection target area WAR has an anomaly. Specifically, in the example illustrated in Fig. 4, for example, a person is assumed to faint and fall due to an acute disease in the individual room and lie on the flow line between the door DR and the furniture (the desk 41, the chair 42, or the shelf 43). In the example illustrated in Fig. 5, for example, the patient is assumed to fall off the bed 5 in the sick room and lie beside the bed 5.

In the present disclosure, if the determiner 24 determines that the living body present in the detection target area WAR has an anomaly, the determiner 24 detects the event as the occurrence of a fall of the person in the detection target area WAR. In response to the detection of the occurrence of a fall of the person in the detection target area WAR, the determiner 24 reports an alarm indicating the occurrence of a fall of the person in the detection target area WAR to the external apparatus (step S112) and terminates the fall detection process illustrated in Fig. 6.

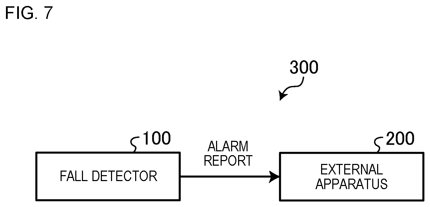

Fig. 7 is a block diagram illustrating an example of a fall detection system. A fall detection system 300 according to an embodiment includes the fall detector 100 described above and an external apparatus 200.

The fall detector 100 and the external apparatus 200 are communicatively configured. A communication medium between the fall detector 100 and the external apparatus 200 may be a wireless communication means defined by a communication standard such as 4G (a fourth-generation mobile communication system), 5G (a fifth-generation mobile communication system), long term evolution (LTE)-frequency division duplex (FDD), LTE-time division duplex (TDD), LTE-Advanced or LTE-Advanced Pro, and the fall detector 100 and the external apparatus 200 may be connected in a wired manner.

The external apparatus 200 may, for example, display an anomaly on a monitor (not illustrated) provided in a room different from the individual room (see Fig. 4) or the sick room (see Fig. 5) where the fall detector 100 is installed or may emit an alarm sound from a speaker (not illustrated). The external apparatus 200 may be a mobile terminal device such as a smartphone of a person to be watched with the fall detector 100.

In the fall detection process described above, the area setting process is executed in a state where a living body is not present in the detection target area WAR.

Specifically, if the statistical signal-strength-variation value PV(WAR) of the detection target area WAR is less than the first threshold PVth1 (No in step S104), the fall detector 100 determines that a moving living body is not present in the detection target area WAR and executes the area setting process (step S105). If the statistical signal-strength-variation value PV(WAR) of the detection target area WAR is greater than or equal to the first threshold PVth1 (Yes in step S104), the living-body-presence determination process for each of the first area PAR1 and the second area PAR2 is executed.

For example, even if the installation location of the radar 1 or the fall det*e*ctor 100 including the radar 1 is changed, the living-body-presence determination process for each of the first area PAR1 and the second area PAR2 that are set appropriately may thereby be executed.

In the fall detection process described above, an area where a person is assumed not to lie under normal conditions is set as the first area PAR1, and the occurrence of a fall of a living body in the detection target area WAR is determined based on a statistical value such as the maximum value or the mean value of the respective signal strength variations PVbin of the plurality of range bins BIN in the first area PAR1.

Specifically, in the process for determining the presence of a living body in the first area PAR1, the fall detector 100 sets, as the first signal strength variation PV(PAR1), the statistical value such as the maximum value or the mean value of the respective signal strength variations PVbin of the plurality of range bins BIN in the first area PAR1 (step S109). If the first signal strength variation PV(PAR1) of the first area PAR1 is greater than or equal to the third threshold PVth3 for determining whether a living body is present in the first area PAR1 (Yes in step S110), and if the first signal strength variation PV(PAR1) of the first area PAR1 is less than or equal to the fourth threshold PVth4 for determining whether the living body present in the detection target area WAR has an anomaly (Yes in step S111), the fall detector 100 determines that the living body present in the detection target area WAR has an anomaly and detects the event as the occurrence of a fall of the person in the detection target area WAR.

The computational load may thereby be reduced more than, for example, in a form where the anomaly on the living body is detected by performing the threshold determination on the signal strength variation PVbin of each of the plurality of range bins BIN in the detection target area WAR.

For example, in the area setting process, a machine learning model may be generated from the signal strength distribution of the detection target area WAR by using classification data for the first area PAR1 and the second area PAR2 as a teacher label of each range bin, and areas as the first area PAR1 and the second area PAR2 may be set for each range bin by using, as input, the acquired signal strength distribution of the detection target area WAR.

For example, in the living-body-presence determination process, a machine learning model may be generated from the signal strength variation distribution of the detection target area WAR by using classification data for living-body presence and anomaly occurrence as a teacher label of each range bin, and the living body presence of the anomaly occurrence may be determined by using, as input, the acquired signal strength variation distribution of the detection target area.

The embodiment described above is provided for easier understanding of the present disclosure and is not intended to limit the interpretation of the present invention. The present disclosure may be changed/improved without departing from the spirit thereof and includes its equivalents.

The present disclosure may have the following configurations as described above or instead of the description above.

(1) A fall detector according to an aspect of the present disclosure includes a sensor; a signal strength calculator that calculates respective signal strengths of a plurality of range bins in a detection target area, based on transmitted and received signals of the sensor; a signal strength variation calculator that calculates respective signal strength variations of the range bins; an area setter that sets a first area in the detection target area, the first area not including a range bin of the range bins that has a corresponding one of the signal strengths that is greater than or equal to a signal strength threshold; and a determiner that detects an occurrence of a fall of a living body in the detection target area based on a first signal strength variation serving as a statistical value of respective signal strength variations of a plurality of range bins in the first area.

With this configuration, the first area that does not include the range bin where the signal strength is greater than or equal to the signal strength threshold is set, and the occurrence of the fall of the living body in the detection target area is detected based on the statistical value of the respective signal strength variations of the plurality of range bins in the first area. The computational load may thereby be reduced.

(2) In the fall detector in (1) above, the sensor is preferably a frequency modulated continuous wave (FMCW) radar or a fast chirp modulation (FCM) radar.

(3) In the fall detector in (1) or (2) above, the area setter sets the first area in response to a statistical signal-strength-variation value being less than a first threshold, the statistical signal-strength-variation value serving as a statistical value of the signal strength variations of the plurality of range bins in the detection target area.

The computational load in setting the first area may thereby be reduced.

(4) In the fall detector in (3) above, the statistical signal-strength-variation value is a maximum value or a mean value of the respective signal strength variations of the plurality of bins in the detection target area.

(5) In the fall detector in (3) above, the first signal strength variation is a maximum value or a mean value of the respective signal strength variations of the plurality of bins in the first area.

(6) In the fall detector in (3) above, the area setter sets a second area excluding the first area.

(7) In the fall detector in (6) above, in response to the statistical signal-strength-variation value being greater than or equal to the first threshold, the determiner detects the occurrence of the fall of the living body in the detection target area, based on the first signal strength variation.

The computational load in detecting the occurrence of the fall of the living body in the detection target area may thereby be reduced.

(8) In the fall detector in (7) above, in response to a second signal strength variation being less than or equal to a second threshold, the second signal strength variation serving as a statistical value of respective signal strength variations of a plurality of range bins in the second area, in response to the first signal strength variation being greater than or equal to a third threshold, and in response to the first signal strength variation being less than or equal to a fourth threshold greater than the third threshold, the determiner determines that the fall of the living body occurs in the detection target area.

The computational load in detecting the occurrence of the fall of the living body in the detection target area may thereby be reduced.

(9) In the fall detector in (8) above, the second signal strength variation is a maximum value or a mean value of the respective signal strength variations of the plurality of bins in the second area.

(10) A fall detection method according to an aspect of the present disclosure includes the fall detector in any one of (1) to (9) above and an external apparatus configured to communicate with the fall detector. The fall detector reports an alarm to the external apparatus, the alarm indicating that the fall occurs in the detection target area.

With this configuration, detection of the occurrence of the fall of the living body in the detection target area may be reported to the external apparatus.

According to the present disclosure, the fall detector and the fall detection method by which the computational load is reduced may be provided.

### Reference Signs List

- 1: radar
- 2: processing unit
- 3: memory
- 5: bed
- 21: signal strength calculator
- 22: signal strength variation calculator
- 23: area setter
- 24: determiner
- 41: desk
- 42: chair
- 43: shelf
- 51: bedside cupboard
- 100: fall detector
- 200: external apparatus
- 300: fall detection system

## Claims

1. A fall detector comprising:
a sensor;
a signal strength calculator that calculates respective signal strengths of a plurality of range bins in a detection target area, based on transmitted and received signals of the sensor;
a signal strength variation calculator that calculates respective signal strength variations of the range bins;
an area setter that sets a first area in the detection target area, the first area not including a range bin of the range bins that has a corresponding one of the signal strengths that is greater than or equal to a signal strength threshold; and
a determiner that detects an occurrence of a fall of a living body in the detection target area based on a first signal strength variation serving as a statistical value of respective signal strength variations of a plurality of range bins in the first area.

2. The fall detector according to claim 1,
wherein the sensor is a frequency modulated continuous wave (FMCW) radar or a fast chirp modulation (FCM) radar.

3. The fall detector according to claim 1 or 2,
wherein the area setter
sets the first area in response to a statistical signal-strength-variation value being less than a first threshold, the statistical signal-strength-variation value serving as a statistical value of the respective signal strength variations of the plurality of range bins in the detection target area.

4. The fall detector according to claim 3,
wherein the statistical signal-strength-variation value is
a maximum value or a mean value of the respective signal strength variations of the plurality of range bins in the detection target area.

5. The fall detector according to claim 3,
wherein the first signal strength variation is
a maximum value or a mean value of the respective signal strength variations of the plurality of range bins in the first area.

6. The fall detector according to claim 3,
wherein the area setter
sets a second area excluding the first area.

7. The fall detector according to claim 6,
wherein in response to the statistical signal-strength-variation value being greater than or equal to the first threshold,
the determiner detects the occurrence of the fall of the living body in the detection target area, based on the first signal strength variation.

8. The fall detector according to claim 7,
wherein in response to a second signal strength variation being less than or equal to a second threshold, the second signal strength variation serving as a statistical value of respective signal strength variations of a plurality of range bins in the second area, in response to the first signal strength variation being greater than or equal to a third threshold, and in response to the first signal strength variation being less than or equal to a fourth threshold greater than the third threshold,
the determiner determines that the fall of the living body occurs in the detection target area.

9. The fall detector according to claim 8,
wherein the second signal strength variation is
a maximum value or a mean value of the respective signal strength variations of the plurality of range bins in the second area.

10. The fall detector according to any one of claims 1 to 9,
wherein the determiner
reports an alarm to an external apparatus, the alarm indicating that the fall occurs in the detection target area.

11. A fall detection method comprising:
calculating respective signal strengths of a plurality of range bins in a detection target area, based on transmitted and received signals of a sensor;
calculating respective signal strength variations of the range bins;
setting a first area in the detection target area, the first area not including a range bin of the range bins that has a corresponding one of the signal strengths that is greater than or equal to a signal strength threshold; and
detecting an occurrence of a fall of a living body in the detection target area, based on a first signal strength variation serving as a statistical value of respective signal strength variations of a plurality of range bins in the first area.

12. The fall detection method according to claim 11,
wherein the sensor is a frequency modulated continuous wave (FMCW) radar or a fast chirp modulation (FCM) radar.

13. The fall detection method according to claim 11 or 12,
wherein in response to a statistical signal-strength-variation value being less than a first threshold, the statistical signal-strength-variation value serving as a statistical value of the respective signal strength variations of the plurality of range bins in the detection target area, the first area is set.

14. The fall detection method according to claim 13,
wherein the statistical signal-strength-variation value is
a maximum value or a mean value of the respective signal strength variations of the plurality of range bins in the detection target area.

15. The fall detection method according to claim 13,
wherein the first signal strength variation is
a maximum value or a mean value of the respective signal strength variations of the plurality of range bins in the first area.

16. The fall detection method according to claim 13, further comprising:
setting a second area excluding the first area.

17. The fall detection method according to claim 16,
wherein in response to the statistical signal-strength-variation value being greater than or equal to the first threshold, the occurrence of the fall of the living body in the detection target area is detected based on the first signal strength variation.

18. The fall detection method according to claim 17,
wherein in response to a second signal strength variation being less than or equal to a second threshold, the second signal strength variation serving as a statistical value of respective signal strength variations of a plurality of range bins in the second area, in response to the first signal strength variation being greater than or equal to a third threshold, and in response to the first signal strength variation being less than or equal to a fourth threshold greater than the third threshold, it is determined that the fall of the living body occurs in the detection target area.

19. The fall detection method according to claim 18,
wherein the second signal strength variation is
a maximum value or a mean value of the respective signal strength variations of the plurality of range bins in the second area.

20. The fall detection method according to any one of claims 11 to 19,
wherein an alarm indicating that the fall occurs in the detection target area is reported to an external apparatus.
